(19) **Europäisches Patentamt**
**European Patent Office**
**Office européen des brevets**

(11) **EP 2 734 141 B1**

(12) # EUROPÄISCHE PATENTSCHRIFT

(45) Veröffentlichungstag und Bekanntmachung des Hinweises auf die Patenterteilung:
**31.08.2016 Patentblatt 2016/35**

(51) Int Cl.:
*A61B 46/20* *(2016.01)* *A61L 31/06* *(2006.01)*
*A61L 31/12* *(2006.01)*

(21) Anmeldenummer: **12735556.8**

(22) Anmeldetag: **17.07.2012**

(86) Internationale Anmeldenummer:
**PCT/EP2012/064014**

(87) Internationale Veröffentlichungsnummer:
**WO 2013/011029 (24.01.2013 Gazette 2013/04)**

(54) **INZISIONSFOLIE UND VERFAHREN ZU DEREN HERSTELLUNG**

INSICION DRAPE AND METHOD FOR PRODUCING THE SAME

FEUILLE D'INCISION ET PROCÉDÉ DE PRODUCTION DE LADITE FEUILLE D'INCISION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.07.2011 DE 102011107637**

(43) Veröffentlichungstag der Anmeldung:
**28.05.2014 Patentblatt 2014/22**

(73) Patentinhaber: **Neschen AG**
**31675 Bückeburg (DE)**

(72) Erfinder:
• **DIETERICH, Norbert**
**30559 Hannover (DE)**

• **HOPPE, Karl**
**31515 Wunsdorf (DE)**
• **MARKIEWICZ, Werner**
**31675 Bückeburg (DE)**

(74) Vertreter: **Uexküll & Stolberg**
**Partnerschaft von**
**Patent- und Rechtsanwälten mbB**
**Beselerstraße 4**
**22607 Hamburg (DE)**

(56) Entgegenhaltungen:
**EP-A1- 0 011 471      EP-B1- 0 677 989**
**WO-A1-03/024494      WO-A2-2008/011024**
**DE-T2- 3 877 135**

EP 2 734 141 B1

**Beschreibung**

Die Erfindung betrifft eine Inzisionsfolie. Weiterhin betrifft die Erfindung ein Verfahren zur Herstellung von Inzisionsfolien.

Inzisionsfolien sind selbstklebende Kunststofffolien, die in der Chirurgie verwendet werden. Sie dienen der sterilen Abdeckung des Operationsfeldes, d.h. der Hautbereiche, in denen chirurgische Eingriffe vorgenommen werden. Eine Inzisionsfolie soll bakteriendicht sein, so dass von außen keine Keime durch die Folie dringen können. Üblicherweise sind Inzisionsfolien wasserdampfdurchlässig. Die Wasserdampfdurchlässigkeit der Folie beugt Feuchtigkeitsansammlungen und Hautmazerationen vor und sorgt zudem für eine sichere Haftung der selbstklebenden Kunststofffolie.

Inzisionsfolien sind im Handel erhältlich. Beispielsweise wird von Smith & Nephew eine klebende transparente Polyurethan-Inzisionsfolie unter der Bezeichnung "OPSITE™" angeboten.

Ein Risiko bei chirurgischen Operationen sind Keime, die sich zwischen der Hautoberfläche und dem Kleber der Inzisionsfolie befinden. Wenn die Inzisionsfolie zusammen mit der Haut während der Operation durchtrennt wird, können die Keime in die Wunde eindringen und zu Infektionen führen. Diese Gefahr ist besonders groß, wenn es sich bei den Keimen um MRSA (Methicillin-resistenter *Staphylococcus aureus*)handelt.

Die Firma 3M™ bietet unter der Bezeichnung "3M™ Ioban™ 2" antimikrobielle Inzisionsfolien an. Diese sollen sowohl einen physikalischen als auch einen chemischen Schutz vor bakteriellen Kontaminationen bieten. Diese bekannte Inzisionsfolie enthält ein Iodophor, das kontinuierlich seine Wirkung während des chirurgischen Eingriffs freisetzt. Bei dieser Inzisionsfolie ist das Iodophor in dem Klebstoff der selbstklebenden Folie enthalten. Die Folie wird als wasserdampfdurchlässig und atmungsaktiv beschrieben. Sie soll anpassungsfähig an Körperkonturen sein. Durch geringe Rückstellkräfte sollen Mobilisation und schwere Retraktion möglich sein. Die Haut werde nicht durch Spannung oder Scherkräfte belastet.

Wie bereits angesprochen, wird bei einer Operation die Inzisionsfolie zusammen mit der Haut vom Chirurgen durchschnitten. Wenn der Klebstoff der Inzisionsfolie ein antimikrobielles Mittel wie beispielsweise ein Iodophor enthält, wird dieses während der Operation langsam freigesetzt und kontinuierlich an die Haut und insbesondere auch in den Wundbereich abgegeben. Eine solche Abgabe eines antimikrobiell wirksamen Mittels in den Wundbereich ist nicht in jedem Fall wünschenswert.

Es ist bekannt, Kunststoffe mithilfe von Silber antimikrobiell auszurüsten. In der WO 95/20878 wird ein Verfahren zur Herstellung von Kunststoffkörpern beschrieben, bei dem ein Kunststoffrohling, beispielsweise eine Kunststofffolie mit einem antimikrobiell wirksamen Metall und/oder eine antimikrobiell wirksame Metallverbindung mittels eines chemischen oder physikalischen Verfahrens beschichtet wird. Der beschichtete Kunststoffrohling wird danach zerkleinert und/oder eingeschmolzen und anschließend nach einem üblichen Verfahren in die gewünschte Form gebracht. Die WO 95/20878 beschreibt die Bedampfung von Polyurethanfolien mit einer Silberschicht von etwa 10 nm Dicke in einer für die Entspiegelung von optischen Gläsern bestimmten HochvakuumAnlage. Die Folien wurden danach zerkleinert und unter Umrühren bei ca. 240°C eingeschmolzen. Darauf wurden durch Heißpressen Kunststoffkörper hergestellt. Der Silbergehalt der Kunststoffkörper betrug 250 ppm. Aufgrund des Silbergehaltes zeigten die hergestellten Kunststoffkörper antimikrobielle Wirksamkeit gegen Staphylococcus epidermidis, Staphylococcus aureus, Streptococcus faecalis, Escherichia coli, Pseudomonas aeruginosa und Candida albicans.

Unter der Bezeichnung "AQUACEL® Ag" wird von der Firma ConvaTec ein mit Silber imprägnierter, antimikrobieller, saugfähiger Wundverband angeboten. Der Anbieter beschreibt den Verband als eine weiche, sterile, nicht gewebte Kompresse oder Tamponade aus Hydrofiber und ionischem Silber. Das im Verbandmaterial enthaltene Silber töte ein breites Spektrum von Wundbakterien ab. Das Verbandmaterial absorbiere große Menge Wundflüssigkeit und Bakterien und bilde ein weiches, kohäsives Gel, das sich exakt an die Wundoberfläche anpasse und so ein feuchtes Wundheilungsmilieu erzeuge.

Unter der Bezeichnung "SILVERCEL" ist im Handel eine antibakterielle Wundauflage mit Silber erhältlich. Diese wird als Hydroalginat mit Silber beschrieben. Sie besteht aus 51 Gew.-% Calciumalginatfasern, 9 Gew.-% Carboxymethylcellulose und 40 Gew.-% silberbeschichteten Fasern. Die Wundauflage ist indiziert für die Behandlung von mäßig bis stark exsudierenden Wunden unterschiedlicher Tiefe.

Smith & Nephew bietet unter der Bezeichnung "Algisite Ag" einen Alginatverband mit Silber an. Dieser soll die positiven Eigenschaften eines Calciumalginates mit der antimikrobiellen Wirkung des Silbers verbinden. Nach Aufnahme von Wundexsudat bildet dieser Wundverband ein Gel und unterstützt damit die feuchte Wundheilung und bildet eine antimikrobielle Barriere gegen Keime. Der Wundverband hat eine antimikrobielle Wirkung gegen ein breites Spektrum von Wundkeimen wie z.B. Staphylococcus aureus und Pseudomonas aeruginosa. Die antimikrobielle Wirkung soll bis zu sieben Tage lang anhalten.

Die Firma Lohmann & Rausche bietet unter der Bezeichnung "Suprasorb® A + Ag" einen Wundverband an, der in der Lage sein soll, infizierte Wunden zu reinigen. Es handelt sich dabei um einen antimikrobiell ausgerüsteten Calciumalginatverband. Die Wundauflage aus vliesartig vernetzten Calciumalginat-Fasern wird aus natürlichen Braunalgen gewonnen und erhält ihre antimikrobielle Wirkung durch den Zusatz von silberhaltigen Alginatfasern. Der Anbieter gibt an, dass durch den Kontakt mit Wundexsudat freigesetzte Silberionen durch die Gelbildung des Alginats ihre Wirkung direkt an der Wundoberfläche entfalten können. Der Wundverband hat laut Anbieter ein breites antimikrobielles Spektrum und ist laut Anbieter auch gegen MRSA und VRE wirksam.

Ein Verfahren zur Herstellung einer Folie nach der Präambel von Anspruch 1 wird in WO 2008/011024 A2 offenbart.

Eine Inzisionsfolie zur chirurgischen Anwendung nach der Präambel von Anspruch 20 wird in DE 38 77 135 T2 offenbart.

Aufgabe der Erfindung ist die Schaffung einer Inzisionsfolie, die einerseits gute antimikrobielle Wirksamkeit auf ihrer der Haut des Patienten zugewandten Seite aufweist, die andererseits aber nicht oder jedenfalls nicht in nennenswertem Umfang ein antimikrobielles Mittel an die Haut des Patienten oder in den Wundbereich abgibt. Zur Aufgabe gehört auch die Bereitstellung eines Verfahrens zur Herstellung einer solcher Inzisionsfolie.

Die Aufgabe wird gelöst durch ein Verfahren zur Herstellung einer Inzisionsfolie, die eine Kunststofffolie, eine Klebstoffschicht auf einer der beiden Oberflächen der Kunststofffolie und ein von der Klebstoffschicht abziehbares Abdeckpapier auf der Klebstoffschicht umfasst, bei dem

eine als Abdeckpapier verwendete Papierbahn auf einer ihrer beiden Oberflächen mit einer ersten Beschichtung versehen wird,

eine wässrige Dispersion eines Haftklebstoffs bereitgestellt wird,

ein teilchenförmiger Füllstoff bereitgestellt wird, dessen Dichte größer als die Dichte der wässrigen Dispersion ist und dessen Oberfläche zumindest teilweise mit Silber beschichtet ist,

der Füllstoff in die wässrige Dispersion eingemischt wird,

die füllstoffhaltige wässrige Dispersion auf die erste Beschichtung des Abdeckpapiers aufgetragen wird, um eine zweite Beschichtung zu erhalten, in welcher sich der Füllstoff auf der ersten Beschichtung absetzt,

die zweite Beschichtung getrocknet wird, um Wasser aus der zweiten Beschichtung zu entfernen und eine Klebstoffschicht zu erhalten, und

eine Kunststofffolie auf die Klebstoffschicht aufgebracht wird, wobei

das Material für die erste Beschichtung so gewählt wird, dass die Klebkraft zwischen der ersten Beschichtung und der Klebstoffschicht kleiner ist als die Klebkraft zwischen der Kunststofffolie und der Klebstoffschicht sowie die Klebkraft zwischen dem Abdeckpapier und der ersten Beschichtung, so dass man das Abdeckpapier mit der ersten Beschichtung zerstörungsfrei von der Kunststofffolie mit der Klebstoffschicht abziehen kann.

Das erfindungsgemäße Verfahren stellt ein sogenanntes Transferbeschichtungs-Verfahren dar. Die Klebstoffschicht wird zunächst auf dem Abdeckpapier erzeugt und anschließend auf die Trägerfolie übertragen. Dabei kann man sich die Tatsache zunutze machen, dass das Trägerpapier im Allgemeinen eine größere Dimensionsstabilität als die Trägerfolie aufweist, weshalb das Trägerpapier leichter mit Klebstoff beschichtet werden kann.

Weiterhin wird die Aufgabe durch die Bereitstellung einer Inzisionsfolie aus einer Kunststofffolie gelöst, die auf einer ihrer beiden Oberflächen eine Klebstoffschicht aufweist und die dadurch gekennzeichnet ist, dass

in der Klebstoffschicht Füllstoffteilchen derart verteilt sind, dass sie an der von der Kunststofffolie abgewandten Seite der Klebstoffschicht angereichert und teilweise nicht mit Klebstoff bedeckt sind und

die Füllstoffteilchen insbesondere an dem nicht mit Klebstoff bedeckten Teil ihrer Oberfläche mit Silber beschichtet sind.

Die erfindungsgemäße Inzisionsfolie kann insbesondere nach dem erfindungsgemäßen Verfahren hergestellt werden. Die Besonderheit der erfindungsgemäßen Inzisionsfolie besteht darin, dass sich an der Oberfläche der Klebstoffschicht mit Silber beschichtete Füllstoffteilchen befinden. Teile der Silberschicht liegen frei, d.h. sie sind nicht mit Klebstoff bedeckt. Diese freiliegenden kleinen Silberflächen, die gegebenenfalls auch aus der Klebstoffschicht hervorragen, in welche die mit Silber beschichteten Füllstoffteilchen eingebettet sind, statten die der Haut des Patienten zugewandte Seite der Inzisionsfolie mit antimikrobieller Wirksamkeit aus.

Die Schicht aus metallischem Silber ist auf den Feststoffteilchen immobilisiert bzw. an die Feststoffteilchen gebunden. Das so immobilisierte Silber wird im Kontakt mit der Haut des Patienten bzw. mit Körperflüssigkeiten des Patienten wie Schweiß oder Wundexsudat nicht oder jedenfalls nicht in nennenswertem Umfang gelöst und abgegeben. Durch die Verwendung der erfindungsgemäßen Inzisionsfolie bei einer Operation wird der Patient also nicht mit Silberionen oder einem anderen antimikrobiellen Mittel belastet.

Der Schutzumfang der Erfindung wird im Verfahrensanspruch 1 und dem Produktanspruch 20 offenbart. Weitere bevorzugte Ausführungsformen werden in den abhängigen Ansprüchen näher spezifiziert.

Die Erfindung wird anhand von Figuren näher erläutert. Es zeigen:

Figur 1      eine schematische Darstellung einer Beschichtungsanlage zur Durchführung des erfindungsgemäßen Verfahrens; und

Figur 2      eine schematische Darstellung eines Querschnitts durch eine auf die Haut aufgeklebte erfindungsgemäße Inszisionsfolie.

Die Trägerfolie erfüllt eine Schutz- und Barrierefunktion. Gleichzeitig ist sie der Träger für den Kleber, der die Folie auf der Haut verankert.

Die als Trägerfolie eingesetzte Kunststofffolie wird vorzugsweise aus Polyurethanfolien, Polyethylenfolien, Polypropylenfolien und coextrudierten Polyethylen-Polypropylenfolien ausgewählt. Besonders bevorzugt als Trägerfolie für die erfindungsgemäße Inzisionsfolie sind Polyurethanfolien. Insbesondere Polyurethanfolien haben den Vorteil, dass sie in wasserdampfdurchlässiger und atmungsaktiver Form bereitgestellt werden können. Sie passen sich auch sehr gut an die Körperkonturen an. Geeignete Kunststofffolien sind dem Fachmann bekannt und im Handel erhältlich.

Der Kleber muss Eigenschaften wie Wasserdampfdurchlässigkeit, Hautverträglichkeit, hinreichende Klebkraft auf der Haut über die Anwendungsdauer und Sterilisierbarkeit aufweisen.

Der Haftklebstoff für die Klebstoffschicht wird vorzugsweise aus Reinacrylaten, modifizierten Acrylaten, Heißschmelzklebstoffen, UV-vernetzenden Heißschmelz-Acrylaten, Lösemittel-Acrylaten und Lösemittel-Kautschuk-Klebern ausgewählt. Dabei sind unter modifizierten Acrylaten mit Klebharzen gemischte Acrylatklebstoffe zu verstehen. Erfindungsgemäß bevorzugte Haftklebstoffe sind Reinacrylate und modifizierte Acrylate.

Derartige Haftklebstoffe sind dem Fachmann bekannt und im Handel erhältlich. Ein Beispiel für einen erfindungsgemäß geeigneten Haftklebstoff ist "Roderm 580", erhältlich von Dow Deutschland, Am Kronberger Hang 4, 65824 Schwalbach, Deutschland.

Als Transportschutz und zur Erhaltung der Dimension wird bei der erfindungsgemäßen Inzisionsfolie die Kleberschicht mit einem abweisenden Papier (Abdeckpapier) abgedeckt.

Papierbahnen, die bei einem Transferbeschichtungs-Verfahren als Abdeckpapier verwendet werden können, sind dem Fachmann bekannt. Vorzugsweise wird als Abdeckpapier ein Glassine-Papier oder ein Kraft-Liner aus reinen Fasern ohne Altpapieranteil verwendet.

Die Papierbahn wird auf derjenigen ihrer beiden Oberflächen, die später der Klebstoffschicht zugewandt sein wird, mit einer ersten Beschichtung versehen. Diese erste Beschichtung dient der Einstellung der Klebkraft gegenüber der Klebstoffschicht der Inzisionsfolie. Das Material für die erste Beschichtung muss so gewählt werden, dass man das Abdeckpapier mit der ersten Beschichtung zerstörungsfrei von der Trägerfolie mit der Klebstoffschicht abziehen kann. Dazu ist es erforderlich, dass die Klebkraft zwischen der ersten Beschichtung und der Klebstoffschicht kleiner ist als einerseits die Klebkraft zwischen der Kunststofffolie und der Klebstoffschicht sowie andererseits die Klebkraft zwischen dem Abdeckpapier und der ersten Beschichtung. Vorzugsweise ist diese erste Beschichtung ein katalytisch und/oder thermisch vernetztes Silikonmaterial. Bei dem erfindungsgemäßen Herstellungsverfahren wird das Silikon auf die Papierbahn aufgetragen und anschließend vernetzt.

Die Beschichtung aus vernetztem Silikon weist vorzugsweise eine Schichtdicke von 0,5 bis 4 $\mu$m und insbesondere von 1 bis 2 $\mu$m auf.

Bei dem erfindungsgemäßen Verfahren wird vorzugsweise ein teilchenförmiger anorganischer Füllstoff verwendet. Die Füllstoffteilchen der erfindungsgemäßen Inzisionsfolie bestehen somit vorzugsweise aus anorganischem Material. Das anorganische Material ist insbesondere aus Glas, Zeolithen, nicht-zeolithischen Molekularsieben und Zirkoniumphosphat ausgewählt. Grundsätzlich kommen als Füllstoffe anorganische Materialien in Betracht, die porös oder nicht porös sind. Es ist jedoch bevorzugt, dass die anorganischen Materialien in dem Sinne nicht porös sind, sich bei der Beschichtung eine Silberschicht im Wesentlichen auf der äußeren Oberfläche der Füllstoffteilchen bildet und das Silber nicht in nennenswertem Ausmaß in Poren oder innere Kanäle des Füllstoffmaterials eindringen kann.

Zur Erzielung der erfindungsgemäß angestrebten antimikrobiellen Wirkung sind bereits sehr dünne Beschichtungen aus Silber auf den Füllstoffteilchen ausreichend. Da ist bevorzugt, dass der Füllstoff an seiner Oberfläche eine im Wesentlichen monoatomare Silberschicht aufweist. Erfindungsgemäß besonders bevorzugt ist die Verwendung von mit Silber bedampftem Glaspulver als teilchenförmiger Füllstoff. Am meisten bevorzugt ist mit Silber bedampftes Glaspulver, bei dem die Silberschicht eine Dicke von 10 nm oder weniger aufweist.

Das Silber kann auf den teilchenförmigen Füllstoff und insbesondere auf das Glaspulver sowohl mithilfe von chemischen als auch mithilfe von physikalischen Verfahren aufgebracht werden. Erfindungsgemäß bevorzugt ist die Bedampfung des Füllstoffs mit Silber. Für die Bedampfung des Füllstoffs mit einer Silberschicht kann beispielsweise eine auch für die Entspiegelung von optischen Gläsern geeignete HochvakuumAnlage verwendet werden.

Ein Beispiel für ein erfindungsgemäß geeignetes, silberbeschichtetes Glaspulver ist das im Handel erhältliche Produkt "Ionpure WPA". Hersteller ist die Ishizuka Glass Co., Ltd., Advanced Glass Company, 1880 Kwai-cho, Iwakura-City, Aichi, 482-8510 Japan.

Wie bereits angesprochen, zeichnet sich die erfindungsgemäße Inzisionsfolie dadurch aus, dass die mit Silber beschichteten Füllstoffteilchen derart in der Klebstoffschicht verteilt sind, dass sie an der von der Kunststofffolie abgewandten Seite der Klebstoffschicht angereichert und teilweise nicht mit Klebstoff bedeckt sind. Diese besondere Verteilung der Füllstoffteilchen innerhalb der Klebstoffschicht wird bei dem erfindungsgemäßen Verfahren durch die Kombination der Transferbeschichtungstechnik mit der Auswahl eines Füllstoffes erzielt, dessen Dichte größer als die Dichte der wässrigen Dispersion des Haftklebers ist. Die wässrige Dispersion des Haftklebers wird bei der Transferbeschichtung auf die beschichtete Seite des Abdeckpapiers aufgetragen. Aufgrund des Dichteunterschieds zwischen Füllstoffteilchen und wässriger Dispersion sinken die Füllstoffteilchen in der wässrigen Dispersion unter Einfluss der Schwerkraft nach unten. Die Füllstoffteilchen setzen sich auf der beschichteten Seite des Abdeckpapiers ab. Es ist bevorzugt, dass die Füllstoffteilchen eine Dichte im Bereich von 2 bis 3 $g/cm^3$ haben. Bei dieser Dichte ist ein ausreichender Dichteunterschied zur Dispersion des wässrigen Haftklebers gewährleistet.

Damit der Füllstoff in der wässrigen Dispersion des Haftklebers in der beschriebenen Weise absinken kann, darf sich keinesfalls eine stabile Suspension des Füllstoffs in der wässrigen Dispersion ausbilden. Insbesondere darf die Teilchengröße des Füllstoffs nicht so gering sein, dass sich eine stabile Suspension ausbildet.

Zur Beschichtung der als Abdeckpapier verwendeten Papierbahn mit der ersten Beschichtung kann ein Walzenauftragswerk verwendet werden. Derartige Vorrichtungen sind dem Fachmann bekannt. Sie umfassen eine Auftragswalze mit einer strukturierten Oberfläche. Mithilfe der Vertiefungen in der strukturierten Oberfläche wird das aufzutragende Material aufgenommen und anschließend auf das zu beschichtende bahnförmige Material aufgetragen.

Auch die zweite Beschichtung, d.h. die wässrige Dispersion des Haftklebers, kann mithilfe eines Walzenauftragswerks auf die beschichtete Seite des Abdeckpapiers aufgebracht werden.

Die erste Beschichtung auf dem Abdeckpapier ist eine vergleichsweise dünne Beschichtung. Es ist bevorzugt, dass diese Beschichtung eine Schichtdicke von 0,5 bis 4 $\mu$m und vorzugsweise von 1 bis 2 $\mu$m aufweist. Diese Angaben beziehen sich auf die fertige Beschichtung, d.h. im Falle einer Silikonbeschichtung auf die vernetzte Silikonschicht.

In Vergleich zu dieser ersten Beschichtung ist die Klebstoffschicht wesentlich dicker. Sie weist im Allgemeinen ein Schichtdicke von 10 bis 80 $\mu$m, vorzugsweise 20 bis 60 $\mu$m, insbesondere 30 bis 50 $\mu$m und am meisten bevorzugt 35 bis 40 $\mu$m auf. Diese Angaben beziehen sich auf die fertige Klebstoffschicht wie sie in der gebrauchsfertigen Inzisionsfolie vorhanden ist. Die wässrige Dispersion des Haftklebers muss bei dem erfindungsgemäßen Verfahren mit einer entsprechend höheren Schichtdicke aufgetragen werden. Wenn die den beschichteten Füllstoff enthaltende wässrige Dispersion des Haftklebers 50% Wasser enthält, muss die zweite Beschichtung in einer Dicke aufgetragen werden, die doppelt so groß wie die angestrebte Dicke der Klebstoffschicht ist. Die zweite Beschichtung aus der wässrigen Dispersion des Haftklebers, welche außerdem die mit Silber beschichteten Füllstoffteilchen enthält, wird getrocknet. Die getrocknete Schicht stellt die erfindungsgemäße Klebstoffschicht dar. Die zu diesem Zeitpunkt des Transferbeschichtungs-Verfahrens frei liegende Oberfläche der Klebstoffschicht ist im Wesentlichen frei von Füllstoffteilchen. Erfindungsgemäß ist die Schichtdicke der Klebstoffschicht größer als die Teilchengröße der Füllstoffteilchen. Diese sind, wie oben beschrieben, in der wässrigen Dispersion des Haftklebers nach unten gesunken. Aufgrund ihrer geringen Größe ragen sie nicht bis zu der freiliegenden Oberfläche der Klebstoffschicht herauf.

Die Größe der Füllstoffteilchen kann beispielsweise durch Laserbeugung gemessen werden. Die Messung kann nach DIN ISO 13320-1:1999-11 erfolgen. Soweit in der Beschreibung und in den Patentansprüchen Teilchengrößen angegeben werden, sind diese dahingehend zu verstehen, dass es sich um Teilchengrößen gemäß Messung durch Laserbeugung, insbesondere gemäß DIN ISO 13320-1:1999-11 handelt. Ein geeignetes Messgerät ist beispielsweise unter der Bezeichnung "SYMPATEC HELOS (H2023) & SUCELL" im Handel erhältlich.

Die Teilchengröße der Füllstoffteilchen kann durch die Angaben $D_{50\%}$ und $D_{98\%}$ beschrieben werden. Dabei bezeichnet $D_{50\%}$ diejenige Teilchengröße, für die gilt, dass 50% der Teilchen, bezogen auf die Gesamtmasse der Füllstoffteilchen, eine Teilchengröße von $D_{50\%}$ oder weniger haben. In diesem Fall bestehen die anderen 50 Gew.-% des Füllstoffs auf Teilchen mit einer Größe oberhalb von $D_{50\%}$. Weiterhin bezeichnet $D_{98\%}$ diejenige Teilchengröße, für die gilt, dass 98% der Teilchen, bezogen auf die Gesamtmasse der Füllstoffteilchen, eine Teilchengröße von $D_{98\%}$ oder weniger haben. In diesem Fall haben nur noch 2 Gew.-% des Füllstoffs eine Teilchengröße oberhalb von $D_{98\%}$.

Die Füllstoffteilchen haben eine solche mittels Laserbeugung bestimmte Teilchengrößenverteilung, sodass $D_{50\%}$ im Bereich von 1 bis 10 $\mu$m liegt und $D_{98\%}$ höchstens 15 $\mu$m beträgt.

Wenn der Füllstoff die vorstehend genannte bevorzugte Teilchengrößenverteilung aufweist und eine Dichte von 2 bis 3 $g/cm^3$ hat, wird er vorzugsweise in einer solchen Menge verwendet, dass die Inzisionsfolie 0,100 bis 0,250

g Füllstoff je m$^3$, insbesondere 0,150 bis 0,200 g Füllstoff je m$^3$ und am meisten bevorzugt 0,165 bis 0,185 g Füllstoff je m$^3$ enthält. Der Füllstoff mit der vorstehend genannten bevorzugten Teilchengrößenverteilung und einer Dichte von 2 bis 3 g/ m$^3$ wird in die wässrige Dispersion des Haftklebstoffs vorzugsweise in einer solchen Menge eingemischt, dass die Klebstoffschicht 0,05 bis 1,5 Gew.% und vorzugsweise 0,1 bis 0,5 Gew.% Füllstoff, bezogen auf das Gesamtgewicht aus Klebstoff und Füllstoff nach dem Trocknen, enthält.

Die erfindungsgemäße Inzisionsfolie, die eine mehrschichtige Anordnung aus Kunststofffolie, Klebstoffschicht und beschichtetem Abdeckpapier darstellt, kann nach ihrer Herstellung zu einer Rolle aufgewickelt werden. In diesem Fall ist es bevorzugt, dass die Inzisionsfolie derart zu einer Rolle aufgewickelt wird, dass sich die Kunststofffolie auf der Außenseite der Rolle befindet.

Die erfindungsgemäße Inzisionsfolie wird sterilisiert und in sogenannte OP-Kits eingepackt. In dieser Form wird sie beispielsweise an Krankenhäuser vertrieben.

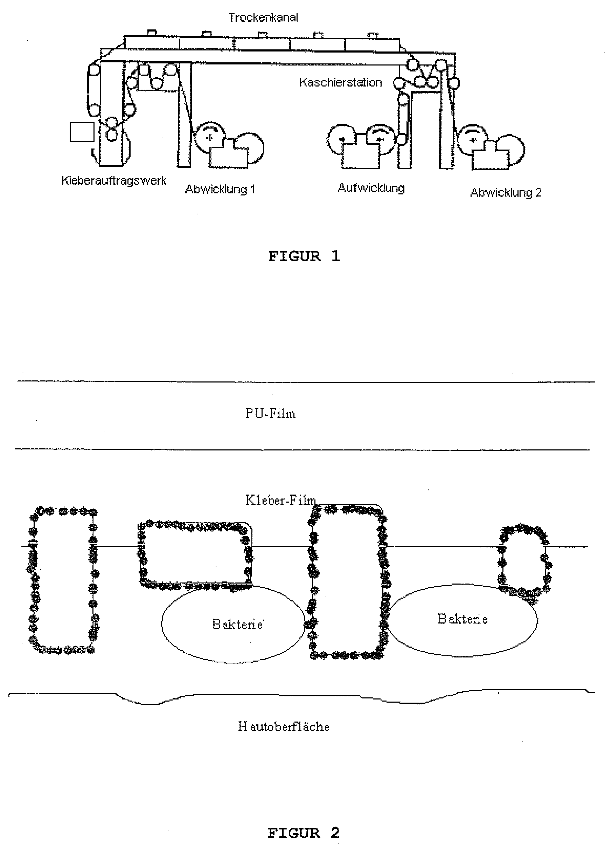

In der in Figur 1 schematisch dargestellten Beschichtungsanlage wird von der Abwicklung 1 silikonisiertes Papier abgewickelt. Im Kleberauftragswerk wird auf die silikonisierte Seite des Papiers die füllstoffhaltige wässrige Dispersion des Haftklebers aufgetragen. Im Trockenkanal setzen sich die silberbeschichteten Füllstoffteilchen auf der Silikonbeschichtung ab, und unter Verdampfen des Wassers bildet sich ein fester Klebstofffilm. Von der Abwicklung 2 wird eine Kunststofffolie abgewickelt. Diese wird in der Kaschierstation auf den Klebstofffilm laminiert. An der Aufwicklung wird die fertige Inzisionsfolie aufgewickelt. Weitere Angaben zur Beschichtungsanlage finden sich in dem nachfolgenden Beispiel.

Die Herstellung des silikonisierten Papiers erfolgt in einer vergleichbaren Anlage. Darin ist das Kleberauftragswerk ersetzt durch ein Mehrwalzenauftragswerk mit dessen Hilfe sehr dünne Schichten von 1 bis 4 μm Schichtdicke aufgetragen werden können. Im Vergleich zu Fig. 1 entfallen in der Anlage für die Herstellung des silikonisierten Papiers die Kaschierstation und die Abwicklung 2. Das mit Silikon beschichtete Papier wird auf sich selbst gewickelt. Die so erhaltene Rolle von silikonisiertem Papier wird anschließend in der Abwicklung 1 der Beschichtungsanlage für die Herstellung der Inzisionsfolie verwendet.

Figur 2 zeigt eine schematische Darstellung eines Querschnitts durch eine auf die Haut aufgeklebte erfindungsgemäße Inzisionsfolie. Von unten nach oben sind gezeigt:

- die Hautoberfläche;

- zwei Bakterien;

- vier im Querschnitt ungefähr rechteckige Füllstoffteilchen, and deren Oberflächen sich (als schwarze Punkte bzw. Sterne dargestellte) Silberatome befinden;

- der Klebstofffilm; und

- der Polyurethanfilm.

Es handelt sich um eine schematische Darstellung, in der insbesondere der Abstand zwischen Kleber-Film und Hautoberfläche stark überhöht dargestellt ist. In der Praxis besteht jedenfalls teilweise direkter Kontakt zwischen Kleber-Film und Hautoberfläche, wenn die erfindungsgemäße Inszisionsfolie auf der Haut des Patienten aufgeklebt wird.

**Beispiel**

Herstellung von silikonisiertem Papier

Die Herstellung von silikonisiertem Papier erfolgte mit einem Mehrwalzenauftragswerk, mit dessen Hilfe sehr dünne Schichten von 1 bis 4 μm aufgebracht werden können. Es wurde ein Glassine-Papier mit einer Stärke von 90 g/m$^2$ verwendet, das von Ahlstrom Turin S.p.A unter der Bezeichnung "Silca Industrial White" erhältlich ist. Als Silikonmasse wurde ein Zweikomponentensystem der Firma Bluestar Silicones Germany verwendet, dessen Komponenten unter den Bezeichnungen "Silcolease Emulsion 902" und "Silcolease Katalysator 903" erhältlich sind. Die beiden Komponenten wurden in einer Inline-Mischanlage gemäß den Angaben des Herstellers gemischt. Die flüssige Silikonmasse wurde in einer Menge von 2 g/m$^2$ auf die Papierbahn aufgetragen und im Trockenkanal der Maschine getrocknet. Bei der Trocknungstemperatur fand die Verdampfung des Wassers und die Vernetzung des Silikons mit dem Papier statt. Die vernetzte Silikonschicht wies eine Dicke von 1 bis 2 μm auf.

Herstellung der Inzisionsfolie

Das silikonisierte Trägerpapier wurde an einer Beschichtungsanlage mithilfe einer Gravurwalze am Kleberauftragswerk mit einer wässrigen Dispersion eines Haftklebers beschichtet. Figur 1 zeigt eine schematische Darstellung der verwendeten Beschichtungsanlage.

Es wurde der Haftkleber "Roderm 580" verwendet, der von Dow Deutschland, Am Kronberger Hang 4, 65824 Schwalbach, Deutschland, erhältlich ist. Dieser Haftkleber ist ein Reinacrylat. Er wird als 56 %ige wässrige Dispersion geliefert.

Als Füllstoff wurde ein silberbeschichtetes Glaspulver verwendet, das unter der Bezeichnung "Ionpure WPA" im Handel erhältlich ist. Dieses silberbeschichtete Glaspulver wird von Ishizuka Glass Co., Ltd., Advanced Glass Company, 1880 Kwai-cho, Iwakura-city, Aichi, 482-8510 Japan, hergestellt. Der Vertrieb in Deutschland erfolgt über die Alfred Kochen GmbH & Co.KG, Poststraße 14-16, 20354 Hamburg. Es werden verschiedenen Teilchengrößen angeboten, die als "<5 $\mu$m", "<10 $\mu$m" und "<50 $\mu$m" bezeichnet werden. Im vorliegenden Beispiel wurde "Ionpure WPA" mit der Teilchengröße "<10 $\mu$m" eingesetzt. $D_{98\%}$ betrug 8,4 $\mu$m, und $D_{50\%}$ betrug 3,0 $\mu$m. Diese Messwerte entsprechen der Spezifikation "<10 $\mu$m". Der Füllstoff hatte eine Dichte von 2,44 g/cm$^3$.

Der Füllstoff wurde der wässrigen Dispersion des Haftklebers in einer Menge von 0,25 Gew.%, bezogen auf das Gesamtgewicht aus Füllstoff und wässriger Dispersion, zudosiert und mithilfe eines Rührers gleichmäßig in die wässrige Dispersion eingemischt. Diese Mischung wurde im Kleberauftragswerk bereitgestellt und kontinuierlich weiter gemischt. Konstruktiv bedingt erfolgte im Auftragswerk ein Rücklauf der wässrigen Dispersion, der um ein Vielfaches höher war als die eigentliche Beschichtungsmenge. Dadurch war ein kontinuierliches Mischen der wässrigen Dispersion gewährleistet.

An einer Station "Abwicklung 1" (Figur 1) wurde silikonisiertes Papier von einer Rolle abgewickelt und über Umlenkrollen zum Kleberauftragswerk geführt. Im Kleberauftragswerk wurde die silikonisierte Seite des Papiers mit der füllstoffhaltigen, wässrigen Dispersion des Haftklebers beschichtet. Das Auftragsgewicht nass betrug 80 g/m$^2$. Die mit der wässrigen Dispersion des Haftklebers beschichtete Papierbahn wurde vom Kleberauftragswerk über Umlenkrollen zum Trockenkanal geführt. Im Trockenkanal befand sich die Papierbahn an der Unterseite und die wässrige Dispersion des Haftklebers an der Oberseite.

Die Geschwindigkeit der mit der wässrigen Dispersion des Haftklebers beschichteten Papierbahn auf ihrem Weg durch den Trockenkanal und das Temperaturprofil im Trockenkanal wurden so gewählt, dass im ersten Abschnitt des Trockenkanals die Füllstoffteilchen innerhalb der wässrigen Dispersion des Haftklebers nach unten sinken und sich auf der silikonisierten Seite des Papiers absetzen konnten. Anschließend wurde das in der wässrigen Dispersion des Haftklebers enthaltene Wasser in den weiteren Abschnitten des Trockenkanals verdampft und es bildete sich ein fester Klebstofffilm. In dem festen Klebstofffilm betrug der Anteil an silberhaltigem Füllstoff etwa 0,5 Gew.%, bezogen auf das Gesamtgewicht des festen Klebstofffilms einschließlich Füllstoff.

Hinter dem Trockenkanal der Maschine befanden sich weitere Auf- und Abwickler und eine Kaschierstation. An der Station "Abwicklung 2" wurde eine Polyurethanfolie mit derselben Geschwindigkeit abgewickelt, mit der die beschichtete Papierbahn durch die Maschine bewegt wurde. Es wurde eine Polyurethanfolie mit einer Dicke von 30 $\mu$m verwendet, die von der Firma Epurex Films GmbH & Co. KG, 29656 Walsrode, Deutschland, erhältlich ist. Die beschichtete Papierbahn wurde vom Ausgang des Trockenkanals und die Polyurethanfolie wurde von der Station "Abwicklung 2" über Umlenkrollen in eine Kaschierstation geführt. Im Kaschierspalt der Kaschierstation wurden beide Bahnen zusammengeführt und über einen geeigneten Anpressdruck miteinander laminiert, in dem die Polyurethanfolie auf die Klebstoffschicht aufgedrückt wurde. Das die Kaschierstation verlassende Laminat hatte die folgende Anordnung von Schichten:

```
PU-Folie/Kleber/Silikon/Papier
```

Über Umlenkrollen wurde das Laminat zur Station "Aufwicklung" geführt. An dieser Station wurde die Folie derart aufgewickelt, dass die Schicht aus Polyurethanfolie die Außenseite der Rolle bildete.

Das aufgewicklelte Laminat stellte die fertige Inzisionsfolie dar. Die Folie zeigte eine gute Klebkraft auf der Haut.

Für die medizinische Verwendung werden von der Rolle Folienstücke abgewickelt und danach abgeschnitten. Die Folienstücke werden sterilisiert und anschließend steril verpackt.

Test auf antimikrobielle Wirksamkeit

Ein Stück der hergestellten Inzisionsfolie wurde auf antimikrobielle Wirksamkeit untersucht. Die Untersuchung wurde gemäß dem japanischen Standard "JIS Z 2801" durchgeführt. Dieser japanische Standard entspricht dem europäischen Standard ISO 22196.

Bei dem Test wurden folgende Bakterien verwendet:

| | |
|---|---|
| Escherichia coli | NBRC 3972 |
| Staphylococcus aureus | NBRC 12732 |

In der nachstehenden Tabelle 1 sind die Testergebnisse für die antimikrobielle Wirkung gegen Escherichia coli gezeigt.

Tabelle 1

| Probe | Anzahl der lebenden Bakterien | | Wert für antimikrobielle Aktivität im Vergleich zum Kontrollversuch | Verminderung der Bakterienzahl in % |
|---|---|---|---|---|
| | am Anfang | nach 24 Stunden | | |
| Erfindungsgemäße Folie | $2.2 \times 10^5$ | $<1 \times 10^2$ | >5.3 | >99.999 |
| Kontrollversuch (Folie ohne Silbergehalt) | $2.2 \times 10^5$ | $2.1 \times 10^7$ | ---- | ---- |

In der nachfolgenden Tabelle 2 sind die Testergebnisse für die antimikrobielle Wirksamkeit gegenüber staphylococcus aureus gezeigt.

Tabelle 2

| Probe | Anzahl der lebenden Bakterien | | Wert für antimikrobielle Aktivität im Vergleich zum Kontrollversuch | Verminderung der Bakterienzahl in % |
|---|---|---|---|---|
| | am Anfang | nach 24 Stunden | | |
| Erfindungsgemäße Folie | $2.9 \times 10^5$ | $<1 \times 10^2$ | >4.0 | >99.999 |
| Kontrollversuch (Folie ohne Silbergehalt) | $2.9 \times 10^5$ | $1.0 \times 10^6$ | ---- | ---- |

Die erfindungsgemäße Folie zeigte eine ausgeprägte antimikrobielle Wirksamkeit. Im Vergleich zum Kontrollversuch zeigte die erfindungsgemäße Folie nach einer Inkubationszeit von 24 Stunden eine Verminderung der Bakterienzahl um mehr als 99,9%.

Extraktionsversuch zur Prüfung auf Silberfreisetzung

Ein 20 cm x 20 cm großes Stück der hergestellten Polyurethan-Inzisionsfolie wurde untersucht um festzustellen, inwieweit die Metalle Aluminium und Silber aus der Inzisionsfolie unter den bei der medizinischen Verwendung herrschenden Bedingungen freigesetzt werden. Zu diesem Zweck wurde das Testmaterial unter Bedingungen, welche den medizinischen Einsatz simulieren, extrahiert. Der Extrakt wurde quantitativ mittels induktiv gekoppelter Massenspektroskopie (ICP-MS) analysiert, um Daten zu erzeugen, die für die Bewertung der biologischen Sicherheit der Inzisionsfolie bei der Verwendung als Medizinprodukt verwendet werden konnten.

Das Folienstück wurde in kleinere Stücke von 4 cm x 4 cm geschnitten, d.h. dass aus dem ursprünglich 20 cm x 20 cm großen Stück 25 kleinere Stücke mit den Abmessungen 4 cm x 4 cm geschnitten wurden. Nach Entfernen des Abdeckpapiers wurden die Probenstücke mit Zellkulturmedium lichtgeschützt 12 Stunden lang bei 37 $\pm$ 1°C extrahiert. Die Extraktion wurde bei einem Oberflächen-Volumen-Verhältnis von 6 cm²/ml durchgeführt, d.h. je 6 cm² Folienfläche wurde 1 ml Extraktionsmedium eingesetzt. Als Reagenzkontrolle wurde das Extraktionsmedium ohne das Testmaterial 12 Stunden bei 37 $\pm$ 1°C inkubiert.

Das verwendete Zellkulturmedium (Dulbecco's modifizietes Eagle Medium, DMEM) enthielt 10 Vol.% fötales Rinderserum (FBS), 100 U/ml Penicillin (P) und 100 μg/ml Streptomycin (S). DMEM (REF FG 1445), FBS (REF S 0615) und P/S (REF A 2213) wurden von Biochrom, Berlin, bezogen.

Der Extrakt und die Reagenzkontrolle wurden mit Salpetersäure aufgeschlossen. Anschließend wurden die freigesetzten Metalle Aluminium und Silber mittels ICP-MS quantifiziert. Die Ergebnisse der Quantifizierungen der metallischen Freisetzungsprodukte sind in der folgenden Tabelle 3 zusammengefasst.

Tabelle 3

| Metall | Extrakt [ng/cm$^2$] | Bestimmungsgrenze [ng/cm$^3$] |
|---|---|---|
| Aluminium | 3.3 | 1.7 |
| Silber | <0.3 | 0.3 |
| (ng/cm$^2$) = ng Element/cm$^2$ Testmaterialoberfläche | | |

Die in der Tabelle 3 gezeigten Werte wurden um die Werte der Reagenzkontrolle korrigiert.

Dieser Versuch zeigt, dass unter den Bedingungen eines medizinischen Einsatzes entweder keine Silberionen freigesetzt werden oder die Freisetzung von Silberionen jedenfalls so gering ist, dass sie unterhalb der Nachweisgrenze der Elementquantifizierung mittels induktiv gekoppelter Plasma-Massenspektroskopie (ICP-MS) liegt.

Der Test wurde entsprechend folgenden Standards durchgeführt:

EN ISO/IEC 17025: 2005, Allgemeine Anforderungen an die Kompetenz von Prüf- und Kalibrierlaboratorien.

DIN EN ISO 10993-1: 2010-04, Biologische Beurteilung von Medizinprodukten - Teil 1: Beurteilung und Prüfungen im Rahmen eines Risikomanagementsystems.

DIN EN ISO 10993-12: 2009-08, Biologische Beurteilung von Medizinprodukten - Teil 12: Probenvorbereitung und Referenzmaterialien.

DIN EN ISO 10993-18: 2009-02, Wasserbeschaffenheit - Anwendung der induktiv gekoppelten Plasma-Massenspektrometrie (ICP-MS) - Teil 18: Chemische Charakterisierung von Werkstoffen

DIN EN ISO 17294-2: 2005-02, Wasserbeschaffenheit - Anwendung der induktiv gekoppelten Plasma-Massenspektrometrie (ICP-MS)- Teil 2: Bestimmung von 62 Elementen

Das Beispiel zeigt, dass die erfindungsgemäße Inzisionsfolie hervorragend antibakteriell wirksam ist ohne ein antibakterielles Mittel in die Wunde des Patienten abzugeben.

**Patentansprüche**

1.  Verfahren zur Herstellung einer Folie, die eine Kunststofffolie, eine Klebstoffschicht auf einer der beiden Oberflächen der Kunststofffolie und ein von der Klebstoffschicht abziehbares Abdeckpapier auf der Klebstoffschicht umfasst, bei dem
    eine als Abdeckpapier verwendete Papierbahn auf einer Ihrer beiden Oberflächen mit einer ersten Beschichtung versehen wird,
    auf die erste Beschichtung ein teilchenförmiger Füllstoff und eine Klebstoffschicht aufgebracht werden und
    eine Kunststofffolie auf die Klebstoffschicht aufgebracht wird, wobei
    das Material für die erste Beschichtung so gewählt wird, dass die Klebkraft zwischen der ersten Beschichtung und der Klebstoffschicht kleiner ist als die Klebkraft zwischen der Kunststofffolie und der Klebstoffschicht sowie die Klebkraft zwischen dem Abdeckpapier und der ersten Beschichtung, so dass man das Abdeckpapier mit der ersten Beschichtung zerstörungsfrei von der Kunststofffolie mit der Klebstoffschicht abziehen kann,
    **dadurch gekennzeichnet, dass**
    die Folie eine Inzisionsfolie zur chirurgischen Anwendung ist, bei deren Herstellung
    eine wässrige Dispersion eines Haftklebstoffs bereitgestellt wird,
    ein teilchenförmiger Füllstoff bereitgestellt wird, dessen Dichte größer als die Dichte der wässrigen Dispersion des Haftklebers ist und dessen Oberfläche zumindest teilweise mit Silber beschichtet ist,
    der Füllstoff in die wässrige Dispersion eingemischt wird,

die füllstoffhaltige wässrige Dispersion auf die erste Beschichtung des Abdeckpapiers aufgetragen wird, um eine zweite Beschichtung zu erhalten, in welcher sich der Füllstoff auf der ersten Beschichtung absetzt, und

die zweite Beschichtung getrocknet wird, um Wasser aus der zweiten Beschichtung zu entfernen und die Klebstoffschicht zu erhalten.

2. Verfahren nach Anspruch 1, **dadurch gekennzeichnet, dass** eine Kunststofffolie verwendet wird, die aus Polyurethanfolien, Polyethylenfolien, Polypropylenfolien und coextrudierten Polyethylen-Polypropylen-Folien ausgewählt ist.

3. Verfahren nach Anspruch 1 oder 2, **dadurch gekennzeichnet, dass** ein Haftklebstoff verwendet wird, der aus Reinacrylaten, modifizierten Acrylaten, Heißschmelzklebstoffen, UV-vernetzenden Heißschmelz-Acrylaten, Lösemittel-Acrylaten und Lösemittel-Kautschuk-Klebern ausgewählt ist.

4. Verfahren nach einem der vorhergehenden Ansprüche, **dadurch gekennzeichnet, dass** auf die Papierbahn eine erste Beschichtung aus einem katalytisch und/oder thermisch vernetzenden Silikon aufgetragen wird, welches anschließend vernetzt wird.

5. Verfahren nach einem der vorhergehenden Ansprüche, **dadurch gekennzeichnet, dass** als Abdeckpapier ein Glassine-Papier oder ein Kraft-liner aus reinen Fasern ohne Altpapieranteil verwendet wird.

6. Verfahren nach einem der vorhergehenden Ansprüche, **dadurch gekennzeichnet, dass** als teilchenförmiger Füllstoff ein anorganischer Füllstoff verwendet wird.

7. Verfahren nach Anspruch 6, **dadurch gekennzeichnet, dass** der anorganische Füllstoff aus Glas, Zeolithen, nicht-zeolithischen Molekularsieben und Zirkoniumphosphat ausgewählt ist.

8. Verfahren nach einem der vorhergehenden Ansprüche, **dadurch gekennzeichnet, dass** ein teilchenförmiger Füllstoff verwendet wird, der mit Silber bedampft worden ist.

9. Verfahren nach einem der vorhergehenden Ansprüche, **dadurch gekennzeichnet, dass** ein teilchenförmiger Füllstoff verwendet wird, der an seiner Oberfläche eine im wesentlichen monoatomare Silberschicht aufweist.

10. Verfahren nach einem der Ansprüche 8 und 9, **dadurch gekennzeichnet, dass** als teilchenförmiger Füllstoff mit Silber bedampftes Glaspulver verwendet wird.

11. Verfahren nach einem der vorhergehenden Ansprüche, **dadurch gekennzeichnet, dass** der Füllstoff mit Silberbeschichtung verwendet wird, der einschließlich der Silberbeschichtung eine Dichte im Bereich von 2 bis 3 $g/cm^3$ hat.

12. Verfahren nach einem der vorhergehenden Ansprüche, **dadurch gekennzeichnet, dass** die erste Beschichtung mit Hilfe eines Walzenauftragswerks auf die Papierbahn aufgebracht wird.

13. Verfahren nach einem der vorhergehenden Ansprüche, **dadurch gekennzeichnet, dass** die zweite Beschichtung mit Hilfe eines Walzenauftragswerks auf die erste Beschichtung des Abdeckpapiers aufgebracht wird.

14. Verfahren nach einem der vorhergehenden Ansprüche, **dadurch gekennzeichnet, dass** eine erste Beschichtung erzeugt wird, die eine Schichtdicke von 0,5 bis 4 $\mu$m und vorzugsweise von 1 bis 2 $\mu$m aufweist.

15. Verfahren nach einem der vorhergehenden Ansprüche, **dadurch gekennzeichnet, dass** eine Klebstoffschicht erzeugt wird, die eine Schichtdicke von 10 bis 80 $\mu$m, vorzugsweise 20 bis 60 $\mu$m, insbesondere 30 bis 50 $\mu$m und am meisten bevorzugt 35 bis 40 $\mu$m aufweist.

16. Verfahren nach einem der vorhergehenden Ansprüche, **dadurch gekennzeichnet, dass** ein teilchenförmiger Füllstoff verwendet wird, der eine solche mittels Laserbeugung bestimmte Teilchengrößenverteilung hat, dass $D_{50\%}$ im Bereich von 1 bis 10 $\mu$m liegt und $D_{98\%}$ höchsten 15 $\mu$m mit der Maßgabe ist, dass $D_{x\%}$ diejenige Teilchengröße ist, für die gilt, dass x% der Teilchen, bezogen auf die Gesamtmasse der Füllstoffteilchen, eine Teilchengröße von $D_{x\%}$ oder weniger haben.

17. Verfahren nach Anspruch 16, **dadurch gekennzeichnet, dass** ein Füllstoff mit einer Dichte von 2 bis 3 $g/cm^3$ in

einer solchen Menge verwendet wird, dass die Inzisionsfolie 0,100 bis 0,250 g Füllstoff je m$^2$ enthält.

18. Verfahren nach Anspruch 16, **dadurch gekennzeichnet, dass** ein Füllstoff mit einer Dichte im Bereich von 2 bis 3 g/cm$^3$ in einer solchen Menge in die wässrige Dispersion des Haftklebstoffs eingemischt wird, dass die Klebstoffschicht 0,05 bis 1,5 Gew.% und vorzugsweise 0,1 bis 0,5 Gew.% Füllstoff, bezogen auf das Gesamtgewicht aus Klebstoff und Füllstoff nach dem Trocknen, enthält.

19. Verfahren nach einem der vorhergehenden Ansprüche, **dadurch gekennzeichnet, dass** die mehrschichtige Anordnung aus Kunststofffolie, Klebstoffschicht und beschichtetem Abdeckpapier derart zu einer Rolle aufgewickelt wird, dass sich die Kunststofffolie auf der Außenseite der Rolle befindet.

20. Inzisionsfolie zur chirurgischen Anwendung aus einer Kunststofffolie, die auf einer ihrer beiden Oberflächen eine Klebstoffschicht aufweist, wobei
in der Klebstoffschicht Füllstoffteilchen derart verteilt sind, dass sie an der von der Kunststofffolie abgewandten Seite der Klebstoffschicht angereichert und teilweise nicht mit Klebstoff bedeckt sind,
die Füllstoffteilchen insbesondere an dem nicht mit Klebstoff bedeckten Teil ihrer Oberfläche mit Silber beschichtet sind und
die Füllstoffteilchen eine solche mittels Laserbeugung bestimmte Teilchengrößenverteilung haben, dass $D_{50\%}$ im Bereich von 1 bis 10 $\mu$m liegt und $D_{98\%}$ höchsten 15 $\mu$m mit der Maßgabe ist, dass $D_{x\%}$ diejenige Teilchengröße ist, für die gilt, dass x% der Teilchen, bezogen auf die Gesamtmasse der Füllstoffteilchen, eine Teilchengröße von $D_{x\%}$ oder weniger haben,
**dadurch gekennzeichnet, dass**
die Inzisionsfolie einen Füllstoff mit einer Dichte im Bereich von 2 bis 3 g/cm$^3$ in einer Menge von 0,100 bis 0,250 g Füllstoff je m$^2$ der Folie enthält.

21. Inzisionsfolie nach Anspruch 20, **dadurch gekennzeichnet dass** die Kunststofffolie aus Polyurethanfolien, Polyethylenfolien, Polypropylenfolien und coextrudierten Polyethylen-Polypropylen-Folien ausgewählt ist.

22. Inzisionsfolie nach Anspruch 20 oder 21, **dadurch gekennzeichnet dass** der Haftklebstoff aus Reinacrylaten, modifizierten Acrylaten, Heißschmelzklebstoffen, UV-vernetzenden Heißschmelz-Acrylaten, Lösemittel-Acrylaten und Lösemittel-Kautschuk-Klebern ausgewählt ist.

23. Inzisionsfolie nach einem der vorhergehenden Ansprüche, **dadurch gekennzeichnet, dass** die Füllstoffteilchen aus anorganischem Material bestehen.

24. Inzisionsfolie nach Anspruch 23, **dadurch gekennzeichnet dass** das anorganische Material aus Glas, Zeolithen, nicht-zeolithischen Molekularsieben und Zirkoniumphosphat ausgewählt ist.

25. Inzisionsfolie nach einem der vorhergehenden Ansprüche, **dadurch gekennzeichnet, dass** die Füllstoffteilchen mit Silber bedampft worden sind.

26. Inzisionsfolie nach einem der vorhergehenden Ansprüche, **dadurch gekennzeichnet, dass** die Füllstoffteilchen auf mindestens einem Teil ihrer Oberfläche eine im wesentlichen monoatomare Silberschicht aufweisen.

27. Inzisionsfolie nach einem der Ansprüche 25 und 26, **dadurch gekennzeichnet, dass** die Füllstoffteilchen Teilchen von mit Silber bedampftem Glaspulver sind.

28. Inzisionsfolie nach einem der vorhergehenden Ansprüche, **dadurch gekennzeichnet, dass** die Klebstoffschicht eine Schichtdicke von 10 bis 80 $\mu$m, vorzugsweise 20 bis 60 $\mu$m, insbesondere 30 bis 50 $\mu$m und am meisten bevorzugt 35 bis 40 $\mu$m aufweist.

29. Inzisionsfolie nach einem der vorhergehenden Ansprüche, **dadurch gekennzeichnet, dass** die Klebstoffschicht mit einem Abdeckpapier abgedeckt ist, welches sich zerstörungsfrei von der Klebstoffschicht abziehen lässt.

30. Inzisionsfolie nach Anspruch 29, **dadurch gekennzeichnet dass** das Abdeckpapier auf seiner der Klebstoffschicht zugewandten Seite eine Beschichtung aus einem katalytisch und/oder thermisch vernetzten Silikon aufweist.

31. Inzisionsfolie nach Anspruch 30, **dadurch gekennzeichnet dass** die Beschichtung aus vernetztem Silikon eine Schichtdicke von 0,5 bis 4 $\mu$m und vorzugsweise von 1 bis 2 $\mu$m aufweist.

**Claims**

1. A method of producing a laminated film, which includes a plastic film, an adhesive layer on one of the two surfaces of the plastic film and a protective paper on the adhesive layer, which is detachable from the adhesive layer, in which a paper web used as the protective paper is provided on one of its two surfaces with a first coating, a particulate filler material and an adhesive layer are applied to the first coating and a plastic film is applied to the adhesive layer, wherein the material for the first coating is so selected that the adhesive force between the first coating and the adhesive layer is smaller than the adhesive force between the plastic film and the adhesive layer and the adhesive force between the protective paper and the first coating so that one can detach the cover paper with the first coating non-destructively from the plastic film with the adhesive layer, **characterised in that** the laminated film is an incision drape for surgical use, in whose production an aqueous dispersion of a contact adhesive is provided, a particulate filler material is provided, the density of which is greater than the density of the aqueous dispersion of the contact adhesive and whose surface is at least partially coated with silver, the filler material is mixed into the aqueous dispersion, the aqueous dispersion containing the filler material is applied to the first coating on the protective paper in order to produce a second coating, in which the filler material precipitates on the first coating, and the second coating is dried in order to remove water from the second coating and to obtain the adhesive layer.

2. A method as claimed in Claim 1, **characterised in that** a plastic film is used, which is selected from polyurethane films, polyethylene films, polypropylene films and coextruded polyethylene-polypropylene films.

3. A method as claimed in Claim 1 or 2, **characterised in that** a contact adhesive is used, which is selected from pure acrylates, modified acrylates, hot melt adhesives, UV-crosslinking hot melt acrylates, solvent acrylates and solvent-rubber adhesives.

4. A method as claimed in one of the preceding claims, **characterised in that** a first coating of catalytically and/or thermally crosslinking silicone is applied to the paper web, which is subsequently crosslinked.

5. A method as claimed in one of the preceding claims, **characterised in that** a glassine paper or a kraft liner of pure fibres without a recovered paper content is used as the protective paper.

6. A method as claimed in one of the preceding claims, **characterised in that** an inorganic filler material is used as the particulate filler material.

7. A method as claimed in Claim 6, **characterised in that** the inorganic filler material is selected from glass, zeolites, non-zeolitic molecular sieves and zirconium phosphate.

8. A method as claimed in one of the preceding claims, **characterised in that** a particulate filler material is used which has been metallised with silver.

9. A method as claimed in one of the preceding claims, **characterised in that** a particulate filler material is used, which has a substantially monoatomic silver layer on its surface.

10. A method as claimed in one of Claims 8 and 9, **characterised in that** glass powder metallised with silver is used as the particulate filler material.

11. A method as claimed in one of the preceding claims, **characterised in that** the filler material with a silver coating is used which, including the silver coating, has a density in the region of 2 to 3 g/cm$^3$.

12. A method as claimed in one of the preceding claims, **characterised in that** the first coating is applied to the paper web with the aid of a roller applicator.

13. A method as claimed in one of the preceding claims, **characterised in that** the second coating is applied to the first coating on the protective paper with the aid of a roller applicator.

14. A method as claimed in one of the preceding claims, **characterised in that** a first coating is produced, which has a layer density of 0.5 to 4 $\mu$m and preferably of 1 to 2 $\mu$m.

15. A method as claimed in one of the preceding claims, **characterised in that** an adhesive layer is produced, which has a layer density of 10 to 80 $\mu$m, preferably 20 to 60 $\mu$m, particularly 30 to 50 $\mu$m and most preferably 35 to 40 $\mu$m.

16. A method as claimed in one of the preceding claims, **characterised in that** a particulate filler material is used which has such a particle size distribution, determined by means of laser diffraction, that $D_{50\%}$ lies in the range of 1 to 10 $\mu$m and $D_{98\%}$ is at most 15 $\mu$m, provided that $D_{x\%}$ is that particle size for which x% of the particles, with respect to the total mass of the filler material particles, has a particle size of $D_{x\%}$ or less.

17. A method as claimed in Claim 16, **characterised in that** a filler material with a density of 2 to 3 g/cm$^3$ is used in such an amount that the incision drape includes 0.100 to 0.250 g filler material per m$^2$.

18. A method as claimed in Claim 16, **characterised in that** a filler material with a density in the range of 2 to 3 g/cm$^3$ is mixed into the aqueous dispersion of the pressure sensitive adhesive in such an amount that the adhesive layer includes 0.05 to 1.5 wt.% and preferably 0.1 to 0.5 wt.% filler material, with respect to the total weight of adhesive and filler material after drying.

19. A method as claimed in one of the preceding claims, **characterised in that** the laminated arrangement of the plastic film, adhesive layer and coated protective paper is rolled up to form a roll such that the plastic film is located on the outside of the roll.

20. An incision drape for surgical use comprising a plastic film, which has an adhesive layer on one of its two surfaces, wherein
filler material particles are distributed in the adhesive layer such that they are concentrated on the side of the adhesive layer remote from the plastic film and are partially not covered with the adhesive,
the filler material particles, particularly at the portion of their surface which is not covered with adhesive, are coated with silver and
the filler material particles have such a particle size distribution, determined by means of laser diffraction that $D_{50\%}$ is in the range of 1 to 10 $\mu$m and $D_{98\%}$ is at most 15 $\mu$m, provided that $D_{x\%}$ is that particle size for which x% of the particles, with respect to the total mass of the filler particles, have a particle size of $D_{x\%}$ or less,
**characterised in that**
the incision drape includes a filler material with a density in the range of 2 to 3 g/cm$^3$ in an amount of 0.100 to 0.250 g filler material per m$^2$ of the drape.

21. An incision drape as claimed in Claim 20, **characterised in that** the plastic film is selected from polyurethane films, polyethylene films, polypropylene films and coextruded polyethylene-polypropylene films.

22. An incision drape as claimed in Claim 20 or 21, **characterised in that** the contact adhesive is selected from pure acrylates, modified acrylates, hot melt adhesives, UV-crosslinking hot melt acrylates, solvent acrylates and solvent-rubber adhesives.

23. An incision drape as claimed in one of the preceding claims, **characterised in that** the filler material particles consist of inorganic material.

24. An incision drape as claimed in Claim 23, **characterised in that** the inorganic material is selected from glass, zeolites, non-zeolitic molecular sieves and zirconium phosphate.

25. An incision drape as claimed in one of the preceding claims, **characterised in that** the filler material particles have been metallised with silver.

26. An incision drape as claimed in one of the preceding claims, **characterised in that** the filler material particles have a substantially monoatomic silver layer on at least a proportion of their area.

27. An incision drape as claimed in one of Claims 25 and 26, **characterised in that** the filler material particles are particles of glass powder metallised with silver.

28. An incision drape as claimed in one of the preceding claims, **characterised in that** the adhesive layer has a layer thickness of 10 to 80 $\mu$m, preferably 20 to 60 $\mu$m, particularly 30 to 50 $\mu$m and most preferably 35 to 40 $\mu$m.

29. An incision drape as claimed in one of the preceding claims, **characterised in that** the adhesive layer is covered with a protective paper, which may be non-destructively detached from the adhesive layer.

30. An incision drape as claimed in Claim 29, **characterised in that** the protective paper has a coating of a catalytically and/or thermally crosslinked silicone on its side directed towards the adhesive layer.

31. An incision drape as claimed in Claim 30, **characterised in that** the coating of crosslinked silicone has a layer thickness of 0.5 to 4 $\mu$m and preferably 1 to 2 $\mu$m.

**Revendications**

1. Procédé pour la fabrication d'un film, qui comprend un film de matière plastique, une couche d'adhésif sur une des deux surfaces du film de matière plastique et un papier de recouvrement pouvant être retiré de la couche d'adhésif sur la couche d'adhésif, dans lequel
une bande de papier utilisée comme papier de recouvrement est munie, sur une de ses deux surfaces, d'un premier revêtement,
sur le premier revêtement, sont appliqués une première charge particulaire et une couche d'adhésif, et
un film de matière plastique est appliqué sur la couche d'adhésif,
le matériau pour le premier revêtement étant choisi de façon à ce que la force d'adhérence entre le premier revêtement et la couche de matière plastique est inférieure à la force d'adhérence entre le film de matière plastique et la couche d'adhésif ainsi que la force d'adhérence entre le papier de recouvrement et le premier revêtement, de façon à ce qu'il soit possible de retirer le papier de recouvrement avec le premier revêtement, sans le détruire, du film de matière plastique avec la couche d'adhésif,
**caractérisé en ce que**
le film est un film d'incision à utilisation chirurgicale, lors de la fabrication duquel une dispersion aqueuse d'adhésif est préparée,
une charge particulaire est préparée, dont la densité est supérieure à la densité de la dispersion aqueuse de l'adhésif et dont la surface est au moins partiellement revêtue d'argent,
la charge est intégrée à la dispersion aqueuse,
la dispersion aqueuse contenant la charge est appliquée sur le premier revêtement du papier de recouvrement, afin d'obtenir un deuxième revêtement dans lequel la charge se dépose sur le premier revêtement, et
le deuxième revêtement est séché afin d'éliminer l'eau du deuxième revêtement et d'obtenir la couche d'adhésif.

2. Procédé selon la revendication 1, **caractérisé en ce qu'**un film de matière plastique est utilisé, qui est sélectionné parmi des films de polyuréthane, des films de polyéthylène, des films de polypropylène et des films coextrudés de polyéthylène-polypropylène.

3. Procédé selon la revendication 1 ou 2, **caractérisé en ce qu'**un adhésif est utilisé, qui est sélectionné parmi des acrylates purs, des acrylates modifiés, des adhésifs thermofusibles, des acrylates thermofusibles à réticulation aux UV, des acrylates à solvants et des adhésifs aux solvants et au caoutchouc.

4. Procédé selon l'une des revendications précédentes, **caractérisé en ce que**, sur la bande de papier, est appliquée un premier revêtement constitué d'un silicone à réticulation catalytique et/ou thermique, qui est ensuite réticulé.

5. Procédé selon l'une des revendications précédentes, **caractérisé en ce que** le papier de recouvrement utilisé peut être un papier Glassine ou un papier Kraft constitué de fibres pures sans part d'ancien papier.

6. Procédé selon l'une des revendications précédentes, **caractérisé en ce que** la charge particulaire utilisée peut être une charge anorganique.

7. Procédé selon la revendication 6, **caractérisé en ce que** la charge anorganique est sélectionnée parmi du verre, des zéolithes, des tamis moléculaires non zéolithiques et du phosphate de zirconium.

8. Procédé selon l'une des revendications précédentes, **caractérisé en ce qu'**une charge particulaire est utilisée, qui a été revêtue d'argent par dépôt en phase vapeur.

9. Procédé selon l'une des revendications précédentes, **caractérisé en ce qu'**une charge particulaire est utilisée, qui comprend, au niveau de sa surface, une couche d'argent essentiellement mono-atomique.

10. Procédé selon l'une des revendications 8 et 9, **caractérisé en ce que** la charge particulaire utilisée est une poudre de verre revêtu d'argent par dépôt en phase vapeur.

11. Procédé selon l'une des revendications précédentes, **caractérisé en ce que** la charge particulaire avec un revêtement d'argent est utilisée, qui présente, avec le revêtement d'argent, une densité de l'ordre de 2 à 3 g/cm$^3$.

12. Procédé selon l'une des revendications précédentes, **caractérisé en ce que** le premier revêtement est appliqué à l'aide d'un dispositif d'application par rouleaux sur la bande de papier.

13. Procédé selon l'une des revendications précédentes, **caractérisé en ce que** le deuxième revêtement est appliqué à l'aide d'un dispositif d'application par rouleaux sur le premier revêtement du papier de recouvrement.

14. Procédé selon l'une des revendications précédentes, **caractérisé en ce qu'**un premier revêtement est produit, qui présente une épaisseur de couche de 0,5 à 4 $\mu$m et de préférence de 1 à 2 $\mu$m.

15. Procédé selon l'une des revendications précédentes, **caractérisé en ce qu'**une couche d'adhésif est produite, qui présente une épaisseur de couche de 10 à 80 $\mu$m, de préférence de 20 à 60 $\mu$m, plus particulièrement de 30 à 50 $\mu$m et de préférence de 35 à 40 $\mu$m.

16. Procédé selon l'une des revendications précédentes, **caractérisé en ce qu'**une charge particulaire est utilisée, qui présente une répartition des tailles de particules, déterminée à l'aide d'une diffraction laser, telle que $D_{50\%}$ est de l'ordre de 1 à 10 $\mu$m et $D_{98\%}$ est de 15 $\mu$m maximum, $D_{x\%}$ étant la taille de particules pour laquelle x % des particules, par rapport à la masse totale des particules de la charge, présente une taille de particule égale à $D_{x\%}$ ou moins.

17. Procédé selon la revendication 16, **caractérisé en ce qu'**une charge avec une densité de 2 à 3 g/cm$^3$ est utilisée dans une quantité telle que le film d'incision contient 0,100 à 0,250 g de charge par m$^2$.

18. Procédé selon la revendication 16, **caractérisé en ce qu'**une charge avec une densité de 2 à 3 g/cm$^3$ est intégrée dans la dispersion aqueuse de l'adhésif dans une quantité telle que la couche d'adhésif contient de 0,05 à 1,5 % en poids et de préférence de 0,1 à 0,5 % en poids de charge, par rapport au poids total de l'adhésif et de la charge après le séchage.

19. Procédé selon l'une des revendications précédentes, **caractérisé en ce que** la disposition en plusieurs couches, constituée d'un film de matière plastique, d'une couche d'adhésif et d'un papier de recouvrement revêtu est enroulée en un rouleau de façon à ce que le film de matière plastique se trouve sur le côté extérieur du rouleau.

20. Film d'incision à application chirurgicale, constitué d'un film de matière plastique, qui comprend, sur une de ses deux surfaces, une couche d'adhésif,
dans la couche d'adhésif, des particules de charge étant réparties de façon à ce qu'elles soient accumulées au niveau du côté, opposé au film de matière plastique, de la couche d'adhésif et ne soient partiellement pas recouvertes d'adhésif,
les particules de charge, plus particulièrement au niveau de la partie, non recouverte d'adhésif, de leur surface, étant recouvertes d'argent et
les particules de charge présentant une répartition des tailles de particules, déterminée à l'aide d'une diffraction laser, telle que $D_{50\%}$ est de l'ordre de 1 à 10 $\mu$m et $D_{98\%}$ est de 15 $\mu$m maximum, $D_{x\%}$ étant la taille de particules pour laquelle x % des particules, par rapport à la masse totale des particules de la charge, présente une taille de particule égale à $D_{x\%}$ ou moins,
**caractérisé en ce que**
le film d'incision contient une charge avec une densité de l'ordre de 2 à 3 g/cm$^3$ dans une quantité de 0,100 à 0,250 g de charge par m$^2$ de film.

**21.** Film d'incision selon la revendication 20, **caractérisé en ce que** le film de matière plastique est sélectionné parmi des films de polyuréthane, des films de polyéthylène, des films de polypropylène et des films coextrudés de polyéthylène-polypropylène.

**22.** Film d'incision selon la revendication 20 ou 21, **caractérisé en ce qu'**un adhésif est utilisé, qui est sélectionné parmi des acrylates purs, des acrylates modifiés, des adhésifs thermofusibles, des acrylates thermofusibles à réticulation aux UV, des acrylates à solvants et des adhésifs aux solvants et au caoutchouc.

**23.** Film d'incision selon l'une des revendications précédentes, **caractérisé en ce que** les particules de charge sont constituées d'un matériau anorganique.

**24.** Film d'incision selon la revendication 23, **caractérisé en ce que** le matériau anorganique est sélectionné parmi du verre, des zéolithes, des tamis moléculaires non zéolithiques et du phosphate de zirconium.

**25.** Film d'incision selon l'une des revendications précédentes, **caractérisé en ce que** les particules de charge ont été revêtues d'argent à l'aide d'un dépôt en phase vapeur.

**26.** Film d'incision selon l'une des revendications précédentes, **caractérisé en ce que** les particules de charge présentent, sur au moins une partie de leur surface, une couche d'argent essentiellement mono-atomique.

**27.** Film d'incision selon l'une des revendications 25 et 26, **caractérisé en ce que** les particules de charge sont des particules de poudre de verre revêtue d'argent à l'aide d'un dépôt en phase vapeur.

**28.** Film d'incision selon l'une des revendications précédentes, **caractérisé en ce que** la couche d'adhésif présente une épaisseur de couche de 10 à 80 $\mu$m, de préférence de 20 à 60 $\mu$m, plus particulièrement de 30 à 50 $\mu$m et de préférence de 35 à 40 $\mu$m.

**29.** Film d'incision selon l'une des revendications précédentes, **caractérisé en ce que** la couche d'adhésif est recouverte d'un papier de recouvrement qui peut être retiré de la couche d'adhésif sans être détruit.

**30.** Film d'incision selon la revendication 29, **caractérisé en ce que** le papier de recouvrement présente, sur son côté opposé à la couche d'adhésif, un revêtement constitué d'un silicone à réticulation catalytique et/ou thermique.

**31.** Film d'incision selon la revendication 30, **caractérisé en ce que** le revêtement en silicone réticulé présente une épaisseur de couche de 0,5 à 4 $\mu$m et de préférence de 1 à 2 $\mu$m.

Trockenkanal

Kaschierstation

Kleberauftragswerk    Abwicklung 1    Aufwicklung    Abwicklung 2

**FIGUR 1**

PU-Film

Kleber-Film

Bakterie    Bakterie

Hautoberfläche

**FIGUR 2**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9520878 A
- WO 2008011024 A2
- DE 3877135 T2